# EUROPÄISCHE PATENTSCHRIFT

(11) **EP 0 769 532 B1**
(45) Veröffentlichungstag und Bekanntmachung des Hinweises auf die Patenterteilung: **13.03.2002**
(21) Anmeldenummer: 96810656.7
(22) Anmeldetag: 02.10.1996
(51) Int. Cl.: C09B 57/00, C07D 471/06, C09K 11/06, C09B 57/08

(54) **Naphthalinlactamimid-Fluoreszenzfarbstoffe**
Naphtholactamimide fluorescent dyes
Colorants fluorescents de naphtolactamimide

(30) Priorität: 12.10.1995 CH 288295
(43) Veröffentlichungstag der Anmeldung: 23.04.1997
(73) Patentinhaber: Ciba Specialty Chemicals Holding Inc., 4057 Basel (CH)
(72) Erfinder: Langhals, Heinz, 85521 Ottobrunn (DE); Von Unold, Petra Christa, 80638 München (DE)

(56) Entgegenhaltungen:
- EP-A- 0 039 085
- DE-A- 1 694 445
- FR-A- 2 253 794
- GB-A- 1 495 296
- ANGEWANDTE CHEMIE. INTERNATIONAL EDITION. , Bd. 34, Nr. 20, 3.November 1995, WEINHEIM.; DE , Seiten 2234-2236, XP002023157 H. LANGHALS ET AL : "tetracarboxylic bisimide -lactam ring contraction: a novel type of rearrangement"
- CHEMICAL ABSTRACTS, vol. 107, no. 5, 3.August 1987 Columbus, Ohio, US; abstract no. 39655v, Seite 686; XP002023158 & ZH. ORG. CHEM., Bd. 22, Nr. 6, 1986, Seiten 1282-1286, BONDARENKO E.F ET AL: "1,4,5,8-Naphthalenetetracarboxylicacid derivatives."
- DATABASE WPI Section Ch, Week 7925 Derwent Publications Ltd., London, GB; Class E23, AN 79-46710B XP002023159 & SU 559 557 A (BONDARENKO E F) , 5.Januar 1979

## Beschreibung

Die vorliegende Erfindung betrifft neue Naphthalin-Lactam-lmide, ein Verfahren zu deren Herstellung aus den entsprechenden Naphthalintetracarbonsäure-Bisimiden, die Bisimide und die verwandten Aminonaphthalintricarbonsäure-lmide als solche, sowie die Verwendung der besagten Verbindungen, beispielsweise zum Färben von hochmolekularem organischen Material.

Perylen-3,4:9,10-tetracarbonsäurebisimide (1) sind als hoch stabile Fluoreszenzfarbstoffe bekannt. Bei den niedrigen Homologen, den Naphthalin-1,8:4,5-tetracarbonsäurebisimiden (2) ist die Absorption so weit kurzwellig verschoben (λ= 380 nm), daß die Substanzen farblos oder nur noch blassgelb sind, so dass sie nicht mehr als Farbstoff eingesetzt werden können (die in der Literatur beschriebenen hellorangenen bis rosa Farbtöne sind auf Verunreinigungen durch Perylenfarbstoffe zurückzuführen). Andererseits wäre es von Interesse, Fluoreszenzfarbstoffe mit Imid-Strukturen auf Naphthalin-Basis zur Verfügung zu haben, da Naphthalin-Derivate im allgemeinen leichter zu synthetisieren und zu reinigen sind als Perylen-Abkömmlinge.

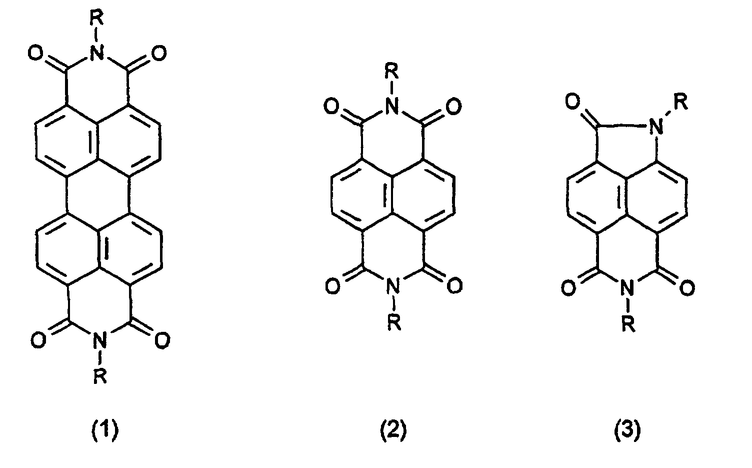

Behandelt man die Naphthalintetracarbonsäure-Bisimide mit KOH in Alkoholen, dann beobachtet man eine erstaunliche Ringverengungsreaktion unter Abspaltung von CO₂ zu den Naphthalin-Imido-Lactamen **3.** Über die Bildung von **3** unter Verwendung von bestimmten Lösungsmitteln ist bereits in J. Org. Chem. USSR, 22(6), 1155 (1986) sowie im SU Patent 559,557 (Derwent Abstract 46710B/25) berichtet worden, und es sind die Derivate mit R = CH₃, -CH₂CH₂CH₂OCH₃, C₆H₅ und 4-BrC₆H₄ dargestellt worden. Diese wenigen vorbeschriebenen Verbindungen sind jedoch als nicht fluoreszierend bekannt.

Nur am Imidstickstoff substituierte Naphthalinlactamimide, worin der Substituent Ethyl, Phenyl, 4-Methoxyphenyl, 3-Chlorphenyl, 3-Chlor-6-methoxyphenyl oder 2,5-Dichlorphenyl bedeutet, sind aus Ind. J. Chem. 24B, 377-382 (1985) bekannt und werden darin als Dispersionsfarbstoffe beschrieben. Auch diese Verbindungen weisen keine Fluoreszenz auf.

### Gegenstand vorliegender Erfindung sind Naphthalin-Lactam-Imide der Formel I

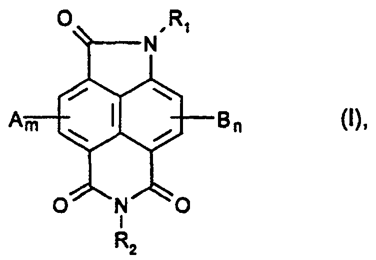

worin
R₁ und R₂ gleich sind, und eine verzweigte unsubstituierte C₃-C₂₅-Alkylgruppe, eine C₂-C₂₅-Alkylgruppe, die mit C₃-C₁₀-Cycloalkyl substituiert ist, oder einen Rest der Formel II 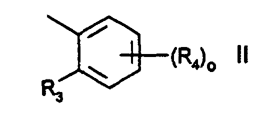 bedeuten, worin R₃ und R₄ C₁-C₁₂-Alkyl sind, und o 0 oder 1 ist.

Bedeuten R₁ und R₂ C₂-C₂₅-Alkyl, so handelt es sich um geradkettige oder vorzugsweise um verzweigte Alkylreste. Bevorzugt sind z.B. 2-Butyl, 2-Methyl-2-butylhexyl, 2,2-Dimethylheptyl oder 2,2-Dihexyloctyl. Durch Cyclohexyl substituiertes Alkyl bedeutet z.B. 1-Ethylcyclohexylmethyl oder 1-n-Propylcyclohexylmethyl.

C₃-C₁₀-Cycloalkyl kann mono- oder auch polycyclisch sein und ist z.B. Cyclopropyl, Cyclobutyl, Cyclopentyl, Cyclohexyl, Cyclooctyl, Cyclodecyl, Bicycloheptyl, Bicyclooctyl oder Decalinyl.

C₁-C₁₂-Alkyl bedeutet z.B. Methyl, Ethyl, n-Propyl, Isopropyl, n-Butyl, sec.-Butyl, tert.-Butyl, Pentyl, tert.-Amyl, Hexyl, und zusätzlich auch z.B. Octyl, 2,2-Dimethylhexyl, Decyl oder Dodecyl.

Besonders geeignete Reste R₁ und R₂ der Formel II sind z.B. 2,3-Dimethylphenyl, 2,5-Dimethylphenyl, 2-Methyl-5-tert.-butylphenyl, 2-tert.-Butylphenyl, 2,3-Di-tert.-butylphenyl oder 2,5-Di-tert.-butylphenyl.

Die erfindungsgemässen Verbindungen der Formel I können z.B. durch Umsetzung von Napthalintetracarbonsäure-Bisimiden der Formel V 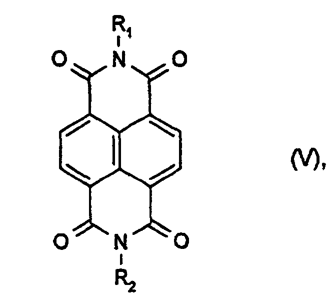 mit einer Base hergestellt werden.

Die Umsetzung erfolgt zweckmässig in einem organischen Lösungsmittel, vorzugsweise in Methanol oder Ethanol. Als Base wird bevorzugt ein Alkalimetallhydroxid, insbesondere Kaliumhydroxid verwendet. Die Gegenwart eines Oxidationsmittels, vorzugsweise Sauerstoff begünstigt die Umsetzung. Von Vorteil ist es auch, wenn das Lösungsmittel Dimethylsulfoxid, zweckmässig 30-50 Vol%, enthält Die Umsetzung findet vorzugsweise bei erhöhter Temperatur, insbesondere zwischen 60 und 120°C statt

Es ist festgestellt worden, dass die obenerwähnte Umsetzung ganz allgemein bei aromatischen Bisimiden stattfindet, insbesondere, wenn unter den erwähnten Vorzugsbedingungen gearbeitet wird.

Naphthalintetracarbonsäure-Bisimide der Formel V sind ebenfalls neue Verbindungen und sind Gegenstand vorliegender Erfindung genauso wie die als Zwischenprodukte entstehenden 1-Aminonaphthalintricarbonsäurederivate der Formel VI, 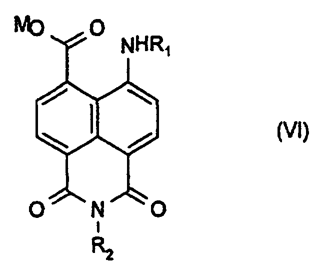 worin R₁ und R₂ die oben angegebene Bedeutung haben, und M für Wasserstoff oder ein Alkalimetallkation, insbesondere für Wasserstoff oder Kalium, steht, mit der Massgabe, dass bei Verbindungen der Formel V R₁ und R₂ nicht beide Ethyl oder Decyl sind.

Die Naphthalintetracarbonsäure-Bisimide der Formel V können auf bekannte Weise, z.B. durch Umsetzung des Bisanhydrids mit dem entsprechenden Amin hergestellt werden. Einzelne, von den erfindungsgemässen Verbindungen der Formel V verschiedene Naphthalintetracarbonsäure-Bisimide sind aus J. Amer. Chem Soc. **89**, 5925 (1967), Synth.
Commun. **19**,1885 (1989) und J. Org. Chem. USSR **13**, 1159 (1977) bekannt.

Wird **2** mit KOH in Ethanol behandelt, dann wird die Reaktion zu **3** überraschenderweise zur Hauptreaktion. Ein Ersatz von Ethanol durch Methanol und Zusatz von DMSO zum Reaktionsgemisch steigert überraschenderweise die Ausbeute von **3** noch weiter - bei aromatischen Substituenten R wird **3** in hohen Ausbeuten erhalten, bei aliphatischen Substituenten sind die Ausbeuten etwas kleiner, aber durchaus akzeptabel.

Für die Darstellung von **3** ist eine Reaktionsführung in Gegenwart von Luftsauerstoff günstig, die Reaktion kann aber auch ohne Sauerstoff durchgeführt werden. Man erhält dann zunächst eine beständige, äußerst Sauerstoff-empfindliche rotviolette Färbung (breite, unstrukturierte Absorption bei 542 nm in einer Mischung aus 2 ml Ethanol, 1 ml DMSO und 0.2 g KOH) der Reaktionslösung und bei der üblichen Aufarbeitung neben **3** als Reaktionsprodukt noch eine kleine Menge des Verseifungs-Produkts eines Imid-Rings, sowie nicht umgesetztes Ausgangsmaterial.

Man beobachtet eine bathochrome Verschiebung der Absorption von **3** gegenüber **2** bis in den sichtbaren Bereich hinein, so daß die Substanzen im Gegensatz zu **2** sehr gut als Farbstoffe für den sichtbaren Bereich verwendet werden können.

Setzt man Bisimide der Formel V als Ausgangsstoffe ein, dann findet man zur Überraschung eine starke Fluoreszenz der Farbstoffe der Formel 1 in Lösung und eine starke Feststoff-Fluoreszenz. Die erfindungsgemässen Verbindungen der Formel I zeichnen sich zudem durch eine ausgezeichnete Lichtechtheit aus und stellen eine neue Klasse von Fluoreszenzfarbstoffen dar. Unerwartet ist auch der große Stokes-Shift der Substanzen, der z.B. bei der Verbindung der Formel I, worin R₁ und R₂ 2,5-Di-tert.-butylphenyl bedeuten, 112 nm beträgt, und die Substanzen für Farbstoff-Laser oder als Signalfarben bzw. für Sicherheitsmarkierungen oder Fluoreszenz-Solarkollektoren interessant macht. Der große Stokes-Shift ist ebenfalls in der Analytik für Fluoreszenzmarkierungen von Interesse. Zum einen läßt sich bei solchen Farbstoffen das Anregungslicht sehr effizient ausblenden und zum anderen lässt sich die Fluoreszenz von störenden Verunreinigungen (mit "normalem" Stokes-Shift) ausblenden, so daß eine hohe analytische Empfindlichkeit und zum anderen ein stabiler, störunempfindlicher Meßwert resultiert.

Bei Verbindungen der Formel I, worin R₁ und R₂ voluminöse Alkyl-Gruppen darstellen, findet man ebenfalls eine starke Fluoreszenz. Diese Substanzen lassen sich wie die bereits erwähnten o-substituierten Aryl-Derivate einsetzen.

Werden schließlich, die Naphthalin-Lactam-lmide der Formel I mit großen Mengen an Alkali behandelt, dann wird der Lactam-Ring unter Erhaltung des Imid-Rings hydrolytisch gespalten, und es resultieren die entsprechenden Salze (die auch bei der beschriebenen Synthese von Verbindungen der Formel I als Zwischenprodukte isoliert werden können). Diese hydrolytische Spaltung ist aber voll reversibel, und die Lactam-Imide der Formel I werden z.B. aus heißer ethanolischer Lösung beim Ansäuern mit konz. Salzsäure oder durch Erhitzen mit Eisessig wieder vollständig zurückerhalten.

Von besonderem Interesse sind Naphthalin-Lactam-lmide der Formel I, worin R₁ und R₂ gleich sind, und vorzugsweise eine verzweigte unsubstituierte C₃-C₂₅-Alkylgruppe, oder ein Rest der Formel II bedeuten, worin R₃ und R₄ C₁-C₁₂-Alkyl sind, und o 0 oder 1 ist, insbesondere aber jene, worin R₁ und R₂ einen Rest der Formel II mit R₃ und R₄ unabhängig voneinander gleich Methyl oder tert.Butyl, vorzugsweise 2-Methylphenyl, 2,3-Dimethylphenyl, 2-Methyl-5-tert.butylphenyl, 2-tert.Butylphenyl oder 2,5-Di-tert.butylphenyl und vor allem diejenigen, worin R₁ und R₂ 2-Butyl, 2-Methyl-2-butylhexyl, 2,2-Dimethylheptyl, 2,2-Dihexyloctyl-, 1-Ethylcyclohexylmethyl oder 1-(n-Propyl)-cycloohexylmethyl bedeuten.

Besonders bevorzugt sind Naphthalin-Lactam-Imide der Formel I, worin R₁ und R₂ -CH(R₈)₂ mit R₈ gleich geradkettiges C₁-C₁₂-Alkyl, vorzugsweise n-Hexyl, n-Heptyl, n-Octyl, n-Nonyl oder n-Decyl sind.

Die erfindungsgemässen Verbindungen eignen sich anhand ihrer Eigenschaften für eine Vielzahl von Anwendungen.

So können sie beispielsweise als Farbstoffe oder als Pigmente für die Massefärbung von Kunststoffen oder Lacken eingesetzt werden. Weitere Gegenstände der Erfindung sind daher in der Masse eingefärbtes hochmolekulares organisches Material enthaltend eine Verbindung der Formel I oder V, sowie ein Verfahren zum Färben von hochmolekularem organischem Material in der Masse unter Verwendung dieser Verbindungen.

Geeignete Kunststoffe sind z.B. Polyolefine, Polyvinylchlorid, Fluorpolymerisate, wie z.B. Polyfluorethylen, Polytrifluorchlorethylen oder Tetrafluorethylen/Hexafluorpropylen-Mischpolymerisat, Silikonharze, insbesondere aber Ingenieurwerkstoffe (Engineering Plastics), wie z.B. Polycarbonate, Polyacrylate, Polymethacrylate, Polystyrol, ABS, Polyester, insbesondere Polyalkylenterephthalate, wie Polybutylenterephthalat (PBT) oder Polyethylenterephthalat (PET), Polyamide, Polyetherketone, Polyurethane, einzeln oder in Mischungen. Zweckmässig werden die Farbmittel (Farbstoffe bzw. Pigmente) in einer Konzentration von 0.01 bis 10, vorzugsweise 0.01-5 Gew%, bezogen auf das Polymer, eingesetzt.

Als Beispiele für Polyolefine, die mit den erfindungsgemässen Verbindungen gefärbt werden können, seien Polyethylen hoher und niederer Dichte (HD-PE, LD-PE und LLD-PE), Polyisobutylen und insbesondere Polypropylen, sowie Copolymere von Polyolefinen mit z.B. Polyethem, Polyetherketonen oder Polyurethanen, erwähnt. Bevorzugt ist Polypropylen.

Die Färbung erfolgt nach den üblichen Verfahren, beispielsweise durch Mischen einer erfindungsgemässen Verbindung oder eines Gemisches solcher Verbindungen mit dem Kunststoffgranulat oder -pulver, ohne es vorher in ein Präparat einarbeiten zu müssen, und Extrudieren der Mischung zu Fasern, Folien oder Granulaten. Letztere können dann beispielsweise im Spritzgussverfahren zu Gegenständen verformt werden.

Die erhaltenen rot fluoreszierenden Ausfärbungen weisen hohe Reinheit und hohe Sättigung auf und zeichnen sich durch gute Transparenz sowie durch gute Beständigkeit, insbesondere gegen Licht aus.

Ein weiterer Gegenstand der Erfindung ist die Verwendung der erfindungsgemässen Verbindungen im Sicherheitsdruck, als Fluoreszenzfarbstoffe für maschinenlesbare Markierungen, als Laserfarbstoffe, sowie für die Herstellung von Druck-Tonern ("non-impact printing toners"), Farbfiltern, organischen Photorezeptoren, Elektrolumineszenz- und Photolumineszenzelementen, optischen Informationsspeichermedien oder Sonnenkollektoren.

Die folgenden Beispiele erläutern die Erfindung.

### BEISPIELE 1-11: HERSTELLUNG DER NAPHTHALINTETRACARBONSAEUREBISIMIDE

### Beipiel 1: N,N'-Di(2-butyl)naphthalin-1,8:4,5-bis(dicarboximid)

2.75 g (10.2 mmol) Naphthalin-1,8:4,5-tetracarbonsäurebisanhydrid werden in 150 ml N,N-Dimethylformamid eingetragen und mit 2.24 g (30.6 mmol) 2-Butylamin versetzt. Das Reaktionsgemisch wird 24 h auf 170°C erhitzt. Danach wird es vorsichtig in 400 ml, auf 0°C abgekühlte, 2N HCI eingerührt. Hierbei fällt ein fleischfarbener Niederschlag aus. Nachdem eine Stunde bei Raumtemperatur gerührt wurde, trennt man das Farbstoff-Rohprodukt über eine D4-Glasfilterfritte ab, verwirft das schwach gelb gefärbte Filtrat und trocknet bei 110°C 8 Stunden. Zur Abtrennung von nicht umgesetztem Edukt wird das sandfarbene, feine Pulver mit 400 ml gesättigter Natriumcarbonatlösung aufgekocht, der unlösliche Rückstand über eine D-4-Glasfilterfritte abgetrennt und erneut bei 110°C 8 Stunden getrocknet. Es werden bei der Umsetzung in N,N'-Dimethylformamid geringe Mengen öliger Nebenprodukte erhalten, die stark am Farbstoff-Rohprodukt anhaften und die vor einer säulenchromatographischen Reinigung abgetrennt werden müssen. Hierzu rührt man das erhaltene Rohprodukt dreimal in je 100 ml Ethanol und filtriert. Das Filtrat wird verworfen. Man erhält 3.82 g (99%) Farbstoff-Rohprodukt, das eine schwache Rosafärbung aufweist. Das Bisimid selbst ist farblos, der Farbeindruck entsteht durch das in Spuren mitentstandene N,N'-Di(2-butyl)perylen-3,4:9,10-bis(dicarboximid), das dünnschichtchromatographisch als einzige Verunreinigung identifiziert wird. Die Abtrennung der Spuren des Perylenbisimides vom Naphthalinbisimid ist sehr schwierig und gelingt auch mit Hilfe der Säulenchromatographie nur schlecht, da die R_{f}-Werte sehr ähnlich sind. Gute Trennergebnisse werden an Kieselgel mit Chloroform/Eisessig im Verhältnis (39: 1) als Laufmittel erzielt; mit Toluol ist ebenfalls eine Trennung möglich. Zur Analysenreinheit mußte das Produkt dreimal säulenchromatographisch gereinigt und anschließend extraktiv aus Ethanol umkristallisiert werden. Ausb. 3.82 g (99 %) - Schmp. 192°C - R_{f}(CHCl₃) = 0.4 - R_{f}(CHCl₃/Eisessig (10:1)) = 0.8. UV(CHCl₃): λₘₐₓ(ε) = 381 (47910), 360 (39040), 343 (22930), 242 (50560).

| | | | | |
|---|---|---|---|---|
| C₄₆H₅₄N₂O₄ (698.9) | Ber. | C 69.83 | H 5.86 | N 7.40 |
| | Gef. | C 69.97 | H 5.93 | N 7.07 |

### Beispiel 2: N,N'-Di(2-methyl-2-butylhexyl)naphthalin-1,8:4,5-bis(dicarboximid)

2.75 g (10.25 mmol) Naphthalin-1,8:4,5-tetracarbonsäurebisanhydrid werden mit 4.38 g (30.6 mmol) 2-Methyl-2-propylhexylamin in 150 ml N,N-Dimethylformamid 24 h zum Sieden erhitzt. Die Lösung wird über Nacht auf Raumtemperatur abgekühlt und mit 400 ml 2N HCI versetzt. Hierbei fällt das Reaktionsprodukt als feiner, beiger Niederschlag an. Zur Vervollständigung der Fällung wird eine Stunde bei Raumtemperatur gerührt. Das Reaktionsprodukt wird über eine D4-Glasfilterfritte von der Reaktionslösung abgetrennt. Man erhält 4.9 g (92%) farbloses Pulver. Zur Elementaranalysenreinheit wird das Produkt säulenchromatographisch an Kieselgel mit Chloroform/Eisessig im Verhältnis 39 : 1 gereinigt. Ausb. 4.7 g (88 %) farbloses Pulver - Schmp. 225°C - R_{f} (Kiesselgel/CHCl₃) = 0.29. - UV (CHCl₃) λₘₐₓ(ε) = 380.7 nm (27660), 360.1 (23170), 342.0 (14650), 326.5 (9690).

| | | | | |
|---|---|---|---|---|
| C₃₂H₄₂N₂O₄ (518.7) | Ber. | C 74.10 | H 8.10 | N 5.40 |
| | Gef. | C 74.16 | H 8.04 | N 5.58 |

### Beispiel 3: N,N'-Di(2,2-dimethylheptyl)naphthalin-1,8:4,5-bis(dicarboximid)

2.75 g (10.3 mmol) Naphthalin-1,8:4,5-tetracarbonsäurebisanhydrid werden mit 4.38 g (30.6 mmol) 2,2-Dimethylheptylamin in 60 ml Eisessig 2.5 h zum Sieden erhitzt. Die Lösung wird über Nacht auf Raumtemperatur abgekühlt und anschließend mit 400 ml 2N HCl versetzt. Hierbei fällt das Reaktionsprodukt als feiner, beiger Niederschlag an. Zur Vervollständigung der Fällung wird eine Stunde bei Raumtemperatur gerührt. Das Reaktionsprodukt wird über eine D-4-Glasfilterfritte von der Reaktionslösung abgetrennt und 8 h bei 110°C getrocknet. Um nicht umgesetztes Bisanhydrid zu entfernen, wird das Rohprodukt zweimal in je 200 ml 10 proz. Kaliumcarbonatlösung aufgekocht, der unlösliche Rückstand über eine D-4-Glasfilterfritte abgetrennt und 8 h bei 110°C getrocknet. Man erhält 2.4 g Rohprodukt (45%, mit bereits sehr hohem Reinheitsgrad). Zur Reinigung wird das Produkt in siedendem Chloroform aufgelöst, geringe Mengen eines unlöslichen, hellgrauen Nebenproduktes (150 mg) abfiltriert und solange vorsichtig Ethanol zugetropft, bis die Kristallisation einsetzt. Man läßt über Nacht stehen und erhält als 1. Fraktion 1.04 g feine, farblose, stark ineinander verfiltzte, schmierig wirkende Nädelchen. Das Filtrat wird eingedampft und die zweite Fraktion: 1.10 g gewonnen. Zur Elementaranalysenreinheit wird die 1. Fraktion säulenchromatographisch an Kieselgel mit Chloroform als Laufmittel gereinigt. Ausb. 1.90 g (35.7%) farbloses Pulver - Schmp. 165°C - R_{f}(CHCl₃) = 0.59. - R_{f}(CHCl₃/Eisessig 39:1) = 0.61. - UV(CHCl₃): λₘₐₓ(ε) = 381 (17160), 361 (16940), 344 (12100), 242 (23280), 327 (7180).

| | | | | |
|---|---|---|---|---|
| C₃₂H₄₂N₂O₄ (518.7) | Ber. | C 74.10 | H 8.16 | N 5.40 |
| | Gef. | C 74.26 | H 8.36 | N 5.30 |

### Beispiel 4: N,N'-Di(2,2-dihexyloctyl)naphthalin-1,8:4,5-bis(dicarboximid)

2.75 g (10.3 mmol) Naphthalin-1,8:4,5-tetracarbonsäurebisanhydrid werden mit 9.11 g (30.6 mmol) 2,2-Dihexyloctylamin in 150 ml N,N-Dimethylformamid 24 h zum Sieden erhitzt. Die Lösung wird über Nacht auf Raumtemperatur abgekühlt, hierbei fällt der Hauptteil des Reaktionsproduktes bereits aus. Der nicht homogene Niederschlag wird abfiltriert, das Filtrat auf 0°C abgekühlt und mit 400 ml 2 N HCI (0°C) versetzt. Hierbei fällt ein geringer Anteil an Produkt aus, der mit dem Hauptanteil an Ausgangsmaterial vereinigt ist. Um nicht umgesetztes Bisanhydrid zu entfernen wird das Rohprodukt einmal in 400 ml 10 proz. Kaliumcarbonatlösung aufgekocht, hierbei schmilzt das Reaktionsprodukt aufgrund seines niedrigen Schmelzpunktes (ca. 50°C). Nachdem auf 0°C abgkühlt wurde, erstarrt das Reaktionsprodukt und läßt sich über eine D-4-Glasfilterfritte abtrennen und wird 8 h im Vakuum über CaCl₂ getrocknet. Das Rohprodukt wird in Chloroform aufgelöst, von einer geringen Menge unlöslichen Nebenproduktes (0.4 g) abfiltriert und zur ElementaranalysenReinheit säulenchromatographisch an Kieselgel mit Chloroform als Laufmittel gereinigt. Ausb. 6.4 g (76%) farbloses Pulver - Schmp. 77°C - R_{f}(CHCl₃) = 0.95. - R_{f}(CHCl₃/Eisessig 39: 1) = 0.90. - UV(CHCl₃): λₘₐₓ(ε) = 381 (29150), 361 (24440), 342 (14810), 327 (9550), 242 (31670).

| | | | | |
|---|---|---|---|---|
| C₅₄H₈₆N₂O₄ (827.3) | Ber. | C 78.40 | H 10.48 | N 3.39 |
| | Gef. | C 77.79 | H 10.18 | N 3.61 |

### Beispiel 5: N,N'-Di(1-ethylcyclohexylmethyl)naphthalin-1,5:4,5-bis(dicarboximid)

2.75 g (10.2 mmol) Naphthalin-1,8:4,5-tetracarbonsäurebisanhydrid werden in 30 ml Eisessig suspendiert und mit 4.33 g (30.6 mmol) 1-Ethyl-1-aminomethylcyclohexan versetzt. Nachdem 15 Minuten zum Sieden erhitzt wurde, ist die Reaktionsmischung aufgrund des ausgefallenen Niederschlages so zäh geworden, daß ein Durchrühren nicht mehr möglich ist. Die Reaktion wird abgebrochen, die Reakionslösung auf Raumtemperatur abgekühlt und der Niederschlag über eine D4-Glasfilterfritte abgetrennt. Nach kurzer Trocknung bei 110°C wird das sandfarbene Pulver mit 300 ml 10 proz. Kaliumcarbonatlösung ausgekocht; hierdurch werden Reste des Anhydrids entfernt. Nach erneuter Trocknung, 8 h bei 110°C, isoliert man 550 mg (10.5%) schwach graues Pulver. Zur Abtrennung geringer Mengen an Nebenprodukten und letzten Edukt-Resten wird eine Säulenchromatographie an Kieselgel mit Chloroform/Eisessig (39:1) als Laufmittel durchgeführt. Ausb. 550 mg (10.5%). Das hochgereinigte Produkt besteht aus farblosen, klebrigen Nadeln - Schmp. 256°C. - R_{f}(CHCl₃) = 0.5.-R_{f}(CHCl₃/Eisessig (10:1)) = 0.87. - UV(CHCl₃): λₘₐₓ(ε) = 382 nm (22580), 361 (19090), 343 (11960) 326(7780), 241 (24380).

| | | | | |
|---|---|---|---|---|
| C₃₂H₃₈N₂O₄ (514.7) | Ber. | C 74.68 | H 7.44 | N 5.44 |
| | Gef. | C 74.82 | H 7.32 | N 5.59 |

### Beispiel 6: N,N'-Di(1-n-propyl-cyclohexylmethyl)naphthalin-1,8:4,5-bis(dicarboximid)

2.75 g (10.2 mmol) Naphthalin-1,8:4,5-tetracarbonsäurebisanhydrid werden in 30 ml Eisessig suspendiert und mit 4.75 g (30.6 mmol) 1-Propyl-1-aminomethylcyclohexan versetzt. Die Reaktionsmischung wird 2 h zum Rückfluß erhitzt, und dann wird die Reaktion durch Abkühlen auf Raumtemperatur abgebrochen. Das Gemisch wird mit 200 ml 2 N HCI versezt. Zur Vervollständigung der Fällung wird 1 h bei Raumtemperatur gerührt. Nach kurzer Trocknung bei 110°C wird das sandfarbene Pulver in 300 ml 10 proz. Kaliumcarbonatlösung ausgekocht, hierdurch werden Reste des Anhydrids entfernt. Nach erneuter Trocknung, 8 h bei 110°C, isoliert man 1.4 g (30%) farbloses Pulver. Zur Abtrennung geringer Mengen an Nebenprodukten und letzten Edukt-Resten wird eine Säulenchromatographie an Kieselgel mit Chloroform als Laufmittel durchgeführt und das Produkt aus Chloroform/Ethanol umkristallisiert. Ausb. 780 mg (15%) farblose, feine Blättchen - Schmp. 227°C - R_{f} (Kieselgel/CHCl₃) = 0.80.

| | | | | |
|---|---|---|---|---|
| C₃₄H₄₂N₂O₄ (542,7) | Ber. | C 75.24 | H 7.80 | N 5.20 |
| | Gef. | C 74.78 | H 7.52 | N 5.22 |

### Beispiel 7: N,N'-Di(2,3-di-methylphenyl)naphthalin-1,8:4,5-bis(dicarboximid)

1.02 g (3.80 mmol) Naphthalin-1,8:4,5-tetracarbonsäurebisanhydrid werden mit 1.37 g (11.31 mmol) 2,3-Dimethylanilin in 20 ml Eisessig 1 h zum Sieden erhitzt, hierbei bildet sich nach anfänglicher Lösung ein voluminöser Niederschlag. Die Reaktionslösung wird abgekühlt und mit 200 ml dest. Wasser versetzt. Der Niederschlag wird abgetrennt und zweimal in 200 ml 10 proz. Kaliumcarbonatlösung aufgekocht um nicht umgesetztes Bisanhydrid zu entfernen. Das Reaktionsprodukt wird über eine D4-Glasfilterfritte abgetrennt und 8 h bei 110°C getrocknet. Man erhält 1.74 g (97 %) Rohprodukt, der Reinheitsgrad ist bereits sehr hoch. Ein Teil des Rohprodukts wird in Chloroform aufgelöst und zur Elementaranalysenreinheit säulenchromatographisch an Kieselgel mit Chloroform als Laufmittel gereinigt. Ausb. 1.10 g (61 %) feine, gelbe Nadeln - Schmp. >350°C - R_{f}(CHCl₃) = 0.25. - R_{f}(CHCl₃/Eisessig 39:1) = 0.57.- UV(CHCl₃): λₘₐₓ(ε) = 380(26560), 360 (23940), 342 (14780), 327 (7430), 242 (30300).

| | | | | |
|---|---|---|---|---|
| C₃₀H₂₂N₂O₄ (474.5) | Ber. | C 75.94 | H 4.67 | N 5.90 |
| | Gef. | C 75.61 | H 4.63 | N 5.76 |

### Beispiel 8: N,N'-Di(5-tert-butylphenyl-2-methyl)naphthalin-1,8:4,5-bis(dicarboximid)

2.75 g (10.3 mmol) Naphthalin-1,8:4,5-tetracarbonsäurebisanhydrid werden mit 5.00 g (30.6 mmol) 2-*tert*-Butyl-2-methylphenylamin in 20 ml Eisessig 1 h zum Sieden erhitzt, hierbei bildet sich nach anfänglicher Lösung ein voluminöser Niederschlag. Die Reaktionslösung wird abgekühlt und mit 200 ml dest. Wasser versetzt. Der Niederschlag wird abgetrennt und in zweimal in 200 ml 10 proz. Kaliumcarbonatlösung aufgekocht um nicht umgesetztes Bisanhydrid zu entfernen. Das Reaktionsprodukt wird über eine D4-Glasfilterfritte abgetrennt und 8 h bei 110°C getrocknet. Man erhält 5.10 g (89 %) Rohprodukt, der Reinheitsgrad ist bereits sehr hoch. Ein Teil des Rohproduktes wird in Chloroform aufgelöst und zur Elementaranalysenreinheit säulenchromatographisch an Kieselgel mit Chloroform als Laufmittel gereinigt. Ausb. 3.5 g (61 %, hochgerechnet) feine, gelbe Nadeln - Schmp. 318°C-R_{f}(CHCl₃) = 0.46.- R_{f}(CHCl₃/Eisessig 39:1) = 0.57. - UV(CHCl₃): λₘₐₓ(ε) = 380 (27130), 360 (24410), 343 (14920), 326 (7460), 240 (36720).

| | | | | |
|---|---|---|---|---|
| C₃₆H₃₄N₂O₄ (558.7) | Ber. | C 77.40 | H 6.13 | N 5.01 |
| | Gef. | C 77.69 | H 6.11 | N 5.20 |

### Beispiel 9: N,N'-Di(2-tert-butylphenyl)naphthalin-1,8:4,5-bis(dicarboximid)

2.75 g (10.3 mmol) Naphthalin-1,8:4,5-tetracarbonsäurebisanhydrid werden in 20 ml Eisessig eingetragen und mit 4.57 g (30.6 mmol) 2-tert-Butylanilin versetzt. Das Reaktionsgemisch wird 2 h zum Rückfluß erhitzt. Nach dieser Reaktionszeit wird auf Raumtemperatur abgekühlt und mit 400 ml 2 N HCI versetzt. Der erhaltene Niederschlag wird über eine Porzellannutsche abgetrennt, zur Entfernung von nicht umgesetztem Bisanhydrid mit 300 ml 10 proz. Kaliumcarbonatlösung aufgekocht. Der unlösliche Rückstand wird über eine D4-Glasfilterfritte abgetrennt und 8 h bei 110°C getrocknet. Man erhält 5.33 g (98 %) Farbstoffrohprodukt. Zur Analysenreinheit wird der Farbstoff säulenchromatographisch an Kieselgel mit Chloroform gereinigt, hierbei erhält man zwei dicht aufeinanderfolgende Fraktionen, bei denen es sich um die beiden Atropisomere handelt. Es werden drei Fraktionen abgenommen. Von den erhaltenen Fraktionen wird das Chloroform bis auf wenige Milliliter entfernt und die beiden Produkte durch vorsichtige Ethanol-Zugabe gefällt. Ausb. 4.88 g (90 %) cis und trans in ca. gleichen Anteilen. Ausbeuten: Mischfraktion der Isomeren **I** und **II**: 2.2 g (40.4 %) farbloses Pulver. Isomeres I: 1.4 g (26 %) farbloses Pulver - Schmp. 332 - 338 °C - R_{f}(CHCl₃) = 0.30. - R_{f}(CHCl₃/Eisessig 39:1) = 0.54. - UV(CHCl₃): λₘₐₓ(ε) = 381 (28080), 360 (24290), 343 (14740), 326 (7580), 242 (26140).

| | | | | |
|---|---|---|---|---|
| C₃₄H₃₀N₂O₄ (530.6) | Ber. | C 76.96 | H 5.70 | N 5.28 |
| | Gef. | C 77.34 | H 5.86 | N 4.98 |

Isomeres **II**: 1.28 g (23.5%) farblose feine Nädelchen - Schmp. 333 °C - R_{f}(CHCl₃) = 0.11.-R_{f}(CHCl₃/Eisessig 39:1) = 0.54. - UV(CHCl₃): λₘₐₓ(ε) = 381 (28220), 360 (24690), 342 (14880), 326 (7500), 241 (36560).

| | | | | |
|---|---|---|---|---|
| C₃₄H₃₀N₂O₄ (530.6) | Ber. | C 76.96 | H 5.70 | N 5.28 |
| | Gef. | C 76.55 | H 5.70 | N 5.31 |

### Beispiel 10: N,N'-Di(2,5-di-tert-butylphenyl)naphthalin-1,8:4,5-bis(dicarboximid)

2.75 g (10.2 mmol) Naphthalin-1,8:4,5-tetracarbonsäurebisanhydrid werden in 150 ml N,N-Dimethylformamid eingetragen und mit 3.14 g (30.6 mmol) 2,5-Di-tert-butylanilin versetzt. Das Reaktionsgemisch wird 24 h auf 170°C erhitzt. Das Gemisch wird vorsichtig in 400 ml, auf 0°C abgekühlte, 2N Salzsäure eingerührt. Hierbei fällt ein sandfarbener Niederschlag aus. Nachdem eine Stunde bei Raumtemperatur gerührt wird, trennt man das Farbstoff-Rohprodukt über eine D4-Glasfilterfritte ab, verwirft das schwach gelb gefärbte Filtrat und trocknet bei 110°C 8 Stunden (8.3 g ockerfarbenes Pulver). Zur Abtrennung von nicht umgesetztem Edukt wird das sandfarbene, feine Pulver mit 400 ml gesättigter Natriumcarbonatlösung aufgekocht, der unlösliche Rückstand über eine D4-Glasfilterfritte abgetrennt und erneut bei 110 °C 8 Stunden getrocknet. Es bilden sich bei der Umsetzung in N,N'-Dimethylformamid geringe Mengen öliger Nebenprodukte, die stark am Farbstoff-Rohprodukt anhaften und die vor einer säulenchromatographischen Reinigung abgetrennt werden sollten. Hierzu rührt man das Rohprodukt dreimal in je 100 ml Ethanol und filtriert. Das Filtrat wird verworfen. Man erhält 3.43 g (52%) ockerfarbenes Pulver, das einen schwachen Stich ins Rote aufweist. Das gewünschte Bisimid selbst ist schwach gelb, der Farbeindruck entsteht durch das in Spuren mitentstandene N,N'-Di(2,5-di-*tert*-butyphenyl)perylen-3,4:9,10-bis(dicarboximid), das dünnschichtchromatographisch als einzige Verunreinigung identifiziert wird. Die Abtrennung des Perylenbisimides vom gewünschten Naphthalinbisimid ist sehr schwierig und gelingt auch mit Hilfe der Säulenchromatographie nur schlecht, da die R_{f}-Werte sehr ähnlich sind. Die besten Trennergebnisse werden an Kieselgel mit Chloroform/Eisessig im Verhältnis (39:1) als Laufmittel erzielt. Toluol liefert ein ähnlich gutes Trennergebnis, allerdings ist die Laufgeschwindigkeit sehr gering. Zur Analysenreinheit wird eine geringe Menge des Produktes dreimal säulenchromatographisch gereinigt. Anschließend liefert eine Extraktion mit Ethanol als Lösungsmittel die hochreine Verbindung als schwach gelbe, feine Nädelchen. - Schmp. >250 °C. - R_{f}(CHCl₃) = 0.73.-R_{f}(CHCl₃/Eisessig 39:1) = 0.69. - UV(CHCl₃): λₘₐₓ(ε) = 381 (24980), 360 (22910), 343 (14890), 328 (8400), 242 (40110).

| | | | | |
|---|---|---|---|---|
| C₄₂H₄₆N₂O₄ (642.8) | Ber. | C 78.47 | H 7.21 | N 4.36 |
| | Gef. | C 77.48 | H 7.28 | N 4.32 |

### Beispiel 11: N,N'-Di(2,5-di-tert-butylphenyl)naphthalin-1,8:4,5-bis(dicarboximid)

2.75 g (10.2 mmol) Naphthalin-1,8:4,5-tetracarbonsäurebisanhydrid werden in 60 ml Eisessig eingetragen und mit 4.17 g (40.6 mmol) 2,5-Di-*tert*-butylanilin versetzt. Das Reaktionsgemisch wird 2.0 h zum Rückfluß erhitzt (Ölbadtemperatur: 160°C) und nach dem Abkühlen, vorsichtig in 400 ml 2N Salzsäure eingerührt. Hierbei fällt ein sandfarbener Niederschlag aus. Nachdem eine Stunde bei Raumtemperatur gerührt wurde, trennt man das Farbstoffrohprodukt über eine D4-Glasfilterfritte ab, verwirft das gelbbraun gefärbte Filtrat und trocknet bei 110°C 8 Stunden. Ausb. 8.3 g ockerfarbenes Pulver. Zur Abtrennung von nicht umgesetztem Edukt wird das sandfarbene, feine Pulver mit 400 ml 10 proz. Kaliumcarbonatlösung aufgekocht, der unlösliche Rückstand wird über eine D4-Glasfilterfritte abgetrennt und erneut bei 110°C 8 Stunden getrocknet. Man erhält 8.45 g beigefarbenes Pulver. Zur Analysenreinheit genügt eine säulenchromatographische Reinigung an Kieselgel mit Chloroform als Laufmittel. Anschließend liefert eine Extraktion mit Ethanol als Lösungsmittel Verbindung hochrein als schwach gelbe, feine Nädelchen. Ausb. 3.43 g (52 %) - Schmp. >250°C - R_{f}(CHCl₃) = 0.73. - R_{f}(CHCl₃/Eisessig 39:1) = 0.69. - - UV(CHCl₃): λₘₐₓ(ε) = 381 (24980), 360 (22910), 343 (14890), 328 (8400), 242 (40110).

| | | | | |
|---|---|---|---|---|
| C₄₂H₄₆N₂O₄ (642.8) | Ber. | C 78.47 | H 7.21 | N 4.36 |
| | Gef. | C 77.48 | H 7.28 | N 4.32 |

### BEISPIELE 12-21: HERSTELLUNG DER NAPHTHALINLACTAMIMIDE

### Beispiel 12: N,N'-Di(2-tert-butylphenyl)-1-aminonaphthalin-4,5:8-tricarbonsäure-1,8-lactam-4,5-imid

250 mg (0.50 mmol) des nach Beispiel 9 oben hergestellten N,N'-Di(2-*tert*-butylphenyl)naphthalin-1,8:4,5-bis(dicarboximid) werden mit 260 mg (3.94 mmol) 85 proz. Kaliumhydroxyd (Plätzchen) in 40 ml Ethanol 8 h zum Rückfluß erhitzt. Die anfänglich schwach gelbe Lösung verfärbt sich über Gelb nach Orange und schließlich nach Hellrot. Die Reaktion wird UV/Vis-spektroskopisch verfolgt. Nach 8 h sind die für das Edukt charakteristischen Banden vollständig verschwunden, und es ist nur noch die sehr breite, langwellig verschobene Absorption des Produktes vorhanden. Die Reaktionslösung wird in 200 ml dest. Wasser eingerührt, auf insgesamt 300 ml mit dest. Wasser verdünnt und mit 100 ml konz. HCI versetzt. Hierbei fällt als Reaktionsprodukt ein intensiv oranger Niederschlag an. Zur Vervollständigung der Fällung wird eine Stunde bei Raumtemperatur gerührt und über Nacht stehengelassen. Das Reaktionsprodukt wird über eine D4-Glasfilterfritte von der Reaktionslösung abgetrennt und 8 h bei 110°C getrocknet. Man erhält 300 mg Rohprodukt. Aus dem Dünnschichtchromatogramm (Kieselgel/Chloroform) ist ersichtlich, daß neben der gewünschten Verbindung ein zweites Reaktionsprodukt entsteht, das fest am Kieselgel adsorbiert, am Säulenanfang hängenbleibt. Es handelt sich um N,N'-Di(2-*tert*-butylphenyl)-1-aminonaphthalin-4,5:8-tricarbonsäure-4,5-imid bzw. um das Kaliumsalz dieser ringgeöffneten Verbindung. Das Produkt wird mit 100 ml Ethanol kurz aufgekocht, 15 ml konz. HCI werden zugegeben, es wird bis zum Farbumschlag von Hellrot nach Gelborange erhitzt und die Lösung dann abgekühlt. Nach einer erneuten Fällung mit dest. Wasser und Abtrennung und Trocknung des leuchtend orangen Niederschlags wird das Produkt mit 200 ml 10 proz. Kaliumcarbonatlösung aufgekocht Der unlösliche Rückstand wird über eine Glasfilterfritte abgetrennt und zur Hochreinigung vollständig in Chloroform gelöst und mindestens dreimal säulenchromatographisch an Kieselgel mit Chloroform als Laufmittel gereinigt. Ausb. 160 mg (67%) feines, gelboranges Pulver - Schmp. 247 °C - R_{f}(CHCl₃) = 0.87. UV(CHCl₃): λₘₐₓ(ε) = 431.8 (7000), 361.7 (6330), 343.9 (5240), 314.2 (5780), 265.2 (21750). - Fluoreszenz (CHCl₃): λₘₐₓ = 544.5 nm.

| | | | | |
|---|---|---|---|---|
| C₃₃H₃₀N₂O₃ (502.6) | Ber. | C 78.86 | H 6.02 | N 5.57 |
| | Gef. | C 78.29 | H 5.92 | N 5.36 |

### Beispiel 12 (a): N,N'-Di(2-tert-butylphenyl)-1-aminonaphthalin-4,5:8-tricarbonsäure-4,5-imid-kaliumsalz - Zwischenstufe der Ringverengungsreaktion

240 mg (0.47 mmol) des nach Beispiel 9 oben hergestellten N,N'-Di(2-*tert*-butyl-phenyl)naphthalin-1,8:4,5-bis(dicarboximid) werden wie oben beschrieben mit 700 mg (12.5 mmol) 85 proz. KOH in 10 ml DMSO/Ethanol-Gemisch (2:3) umgesetzt. Nach drei Stunden Reaktionszeit bei 100°C werden 100 ml halbkonzentrierte HCI durch den Rückflußkühler zur noch warmen Reaktionsmischung getropft. Hierbei fällt das Kaliumsalz leuchtend rotorange aus. Zur Vervollständigung der Fällung wird eine Stunde bei Raumtemperatur gerührt, anschließend über eine Glasfilterfritte D4 abgesaugt, das klare Filtrat verworfen, und bei 80°C über Nacht getrocknet. Ausb. 250 mg (96 %) orangerotes Pulver, stark elektrostatisch - R_{f}(CHCl₃) = 0.05. - UV (CHCl₃): λₘₐₓ(ε) = 451.4 nm. - UV (EtOH): λₘₐₓ(ε) = 455.5 nm. - FAB-MS (70 eV): m/z *(%)* = 519.2 (100) [M⁺, C₃₃H₃₁N₂O₄⁻], 520.2 (38), 475.2 (48) [M⁺-CO₂], 476.2 (16).

### Beispiel 13: N,N'-Di(2-tert-butylphenyl)-1-aminonaphthalin-4,5:8-tricarbonsäure-1,8-lactam-4,5-imid - Umsetzung mit KOH in Methanol/DMSO

250 mg (0.50 mmol) des nach Beispiel 11 oben hergestellten N,N'-Di(2-*tert* butylphenyl)naphthalin-1,8:4,5-bis(dicarboximid) werden mit 700 mg (12.5 mmol) 85 proz. Kaliumhydroxyd (Plätzchen) in 6 ml Methanol und 4 ml DMSO 4 h zum Rückfluß erhitzt Die anfänglich schwach gelbe Lösung verfärbt sich über Gelb nach Orange und schließlich nach Hellrot. Die Reaktionslösung wird mit 100 ml halbkonzentrierter Salzsäure versetzt. Hierbei fällt als Reaktionsprodukt ein intensiv oranger Niederschlag an. Zur Vervollständigung der Fällung wird eine Stunde bei Raumtemperatur gerührt und über Nacht stehengelassen. Das Reaktionsprodukt wird über eine D4-Glasfilterfritte von der Reaktionslösung abgetrennt und 8 h bei 110°C getrocknet. Aus dem Dünnschichtchromatogramm (Kieselgel/Chloroform) wird ersichtlich, daß neben der gewünschten Verbindung ein zweites Reaktionsprodukt entsteht, das fest am Kieselgel adsorbiert, am Säulenanfang hängenbleibt. Es handelt sich um N,N'-Di(2-*tert*-butylphenyl)-1-aminonaphthalin-4,5:8-tricarbonsäure-4,5-imid bzw. um das Kaliumsalz dieser ringgeöffneten Verbindung. Das Produkt wird mit 100 ml Ethanol kurz aufgekocht, 15 ml konz. HCI zugesetzt und bis zum Farbumschlag von Hellrot nach Gelborange erhitzt. Nach erneuter Fällung mit dest. Wasser, Abtrennung und Trocknung des leuchtend gelborangen Niederschlags wird das Produkt mit 200 ml 10 proz. Kaliumcarbonatlösung aufgekocht, der unlösliche Rückstand wird über eine Glasfilterfritte abgetrennt, zur Hochreinigung vollständig in Chloroform gelöst und einmal säulenchromatographisch an Kieselgel mit Chloroform als Laufmittel gereinigt. Ausb. 190 mg (79%) feines gelboranges Pulver - Schmp. 247 °C - R_{f}(CHCl₃) = 0.87. - UV(CHCl₃): λₘₐₓ(ε) = 431.8, 361.7, 343.9, 314.2, 265.2. - Fluoreszenz (CHCl₃): λₘₐₓ= 544.5 nm. - MS (70 eV): m/z (%) = 502.2 (1) [M⁺], 487.2 (2) [M⁺-CH₃], 445.2 (100) [M⁺- C(CH₃)₃].

### Beispiel 14: N,N'-Di(2,5-di-tert-butylphenyl)-1-aminonaphthalin-4,5:8-tricarbonsäure-1,8-lactam-4,5-imid - Umsetzung mit KOH in Ethanol

290 mg (0.45 mmol) des nach Beispiel 10 oben erhaltenen N,N'-Di(2,5-di-*tert*-butylphenyl)naphthalin-1,8:4,5-bis(dicarboximid) werden mit 290 mg (4.39 mmol) 85 proz. Kaliumhydroxyd (Plätzchen) in 40 ml Ethanol 8 h unter Rückfluß gekocht Die anfänglich schwach gelbe Lösung verfärbt sich über Gelb nach Orange, Rotbraun und bei Erreichen des Siedepunktes nach Braunschwarz. Die Reaktion wird UV/Vis-spektroskopisch verfolgt. Nach 8 h sind die für das Edukt charakteristischen Banden vollständig verschwunden, und die sehr breite, langwellig verschobene Absorption des Produktes ist vorhanden. Die hellrote Reaktionslösung wird in 200 ml dest. Wasser eingerührt, auf insgesamt 300 ml mit dest. Wasser verdünnt und mit 100 ml konz. HCI versetzt. Man erhält einen intensiv orangen Niederschlag. Zur Vervollständigung der Fällung wird eine Stunde bei Raumtemperatur gerührt. Das Reaktionsprodukt wird über eine D4-Glasfilterfritte von der Reaktionslösung abgetrennt und 8 h bei 110°C getrocknet. Das Produkt wird mit 100 ml Eisessig aufgekocht, erneut mit dest. Wasser gefällt, abgesaugt und 8 h bei 110°C getrocknet. Anschließend wird mit 200 ml 10 proz. Kaliumcarbonatlösung aufgekocht, der unlösliche Rückstand wird abfiltriert und nach erneuter Trocknung das Produkt vollständig in Chloroform gelöst und säulenchromatographisch an Kieselgel mit Chloroform als Laufmittel gereinigt. Ausb. 130 mg (47%) feines oranges Pulver - Schmp. 165-170 °C (mit Bisimid verunreinigtes Produkt)-R_{f}(CHCl₃) = 0.96. - UV(CHCI3): λₘₐₓ(ε) = 433.6 (14410), 381.1 (14300), 362.1 (20920). Fluoreszenz (CHCl₃): λₘₐₓ = 554.8 nm.

| | | | | |
|---|---|---|---|---|
| C₄₁H₄₆N₂O₃ (614.8) | Ber. | C 80.10 | H 7.54 | N 4.56 |
| | Gef. | C 79.93 | H 7.58 | N 4.57 |

### Beispiel 15: N,N'-Di(2,5-di-tert-butylphenyl)-1-aminonaphthalin-4,5:8-tricarbonsäure-1,8-lactam-4,5-imid - Umsetzung mit KOH in Methanol/DMSO

**(a) N,N'-Di(2,5-di-*tert*-butylphenyl)-1-aminonaphthalin-4,5:8-tricarbonsäure-4,5-imidkaliumsalz: -** 590 mg (0.90 mmol) des nach Beispiel 10 oben hergestellten N,N'-Di(2,5-di-*tert*-butylphenyl)naphthalin-1,8:4,5-bis(dicarboximid) werden mit 1.4 g (25 mmol) 85 proz. Kaliumhydroxyd (Plätzchen) in 8 ml Methanol und 6 ml DMSO 3 h auf 100°C erhitzt. Die anfänglich schwach gelbe Lösung verfärbt sich über Gelb nach Orange, Rotbraun und bei Erreichen des Siedepunktes nach Braunschwarz. Die nach beendeter Reaktionszeit hellrote Lösung wird mit 100 ml halbkonz. HCI versetzt Man erhält einen intensiv orangen Niederschlag. Zur Vervollständigung der Fällung wird eine Stunde bei Raumtemperatur gerührt. Das Reaktionsprodukt wird über eine D4-Glasfilterfritte von der Reaktionslösung abgetrennt und 8 h bei 110°C getrocknet. Ausb. 560 mg gelboranges Pulver. - MS(-FAB/70 eV): m/z (%) = 632.2 (61 [M⁺+1], 631.2 (100) [M⁺-2], 588.3 (20) [M⁺-CO₂], 587.3 (46).

**(b) N,N'-Di(2,5-di-*tert*-butylphenyl)-1-aminonaphthalin-4,5:8-tricarbonsäure-4,5-imid** 500 mg N,N'-Di(2,5-di-*tert*-butylphenyl)-1-aminonaphthalin-4,5:8-tricarbonsäure-4,5-imid-kaliumsalz werden mit 50 ml Eisessig aufgekocht, erneut mit 400 ml dest. Wasser gefällt, abgesaugt und 8 h bei 110°C getrocknet. Man erhält 410 mg rotes, feinkristallines Pulver. MS(-FAB/70 eV): m/z (%) = 634.2 (41) [M⁺+1], 633.2 (100) [M⁺], 632.2 (62), 577.1 (23) [M⁺+1-C(CH₃)₃], 576.2 (16) [M⁺-C(CH₃)₃], 575.2 (39).

**(c) N,N'-Di(2,5-di-*tert*-butylphenyl)-1-aminonaphthalin-4,5:8-tricarbonsäure-1,8-lactam-4,5-imid: -** 200 mg (0.32 mmol) N,N'-Di(2,5-di-*tert*-butylphenyl)-1-aminonaphthalin-4,5:8-tricarbonsäure-4,5-imid werden in 100 ml Ethanol zum Sieden erhitzt, 15 ml konz. HCI werden zugegeben, und es wird weiter erhitzt, bis der Farbumschlag von Hellrot nach Gelborange erfolgt ist. Die Lösung wird mit 300 ml dest. Wasser versetzt, es wird 1 h bei Raumtemperatur gerührt und über Nacht stehengelassen. Der gelborange Niederschlag wird über eine Glasfilterfritte (D5) abgesaugt, bei 110°C 8 h getrocknet und an aktiviertem Kieselgel (Akt. II, 10 Gew% dest. H₂O) mit Chloroform chromatographiert. Ausb. 150 mg (79%) feines gelboranges Pulver. Auf den gesamten Ansatz berechnete Ausb. 340 mg (62%) - Schmp. 275 °C - R_{f}(CHCl₃) = 0.96. - UV(CHCl₃): λₘₐₓ(ε) = 432.5 (7970), 361.9 (7450). - Fluoreszenz (CHCl₃): λₘₐₓ = 554.8 nm.

| | | | | |
|---|---|---|---|---|
| C₄₁H₄₆N₂O₃ (614.8) | Ber. | C 80.10 | H 7.54 | N 4.56 |
| | Gef. | C 79.93 | H 7.58 | N 4.57 |

### Beispiel 16: N,N'-Di(2-methyl-5-tert-butylphenyl)-1-aminonaphthalin-4,5:8-tricarbonsäure-1,8-lactam-4,5-imid

190 mg (0.34 mmol) des nach Beispiel 8 oben hergestellten N,N'-Di(2-methyl-5-*tert*-butylphenyl)naphthalin-1,8:4,5-bis(dicarboximid) werden mit 190 mg (2.88 mmol) 85 proz. Kaliumhydroxyd (Plätzchen) in 40 ml Ethanol 8 h zum Rückfluß erhitzt. Die anfänglich schwach gelbe Lösung verfärbt sich über Gelb nach Orange, Rotbraun und bei Erreichen des Siedepunktes nach Braunschwarz. Die Reaktion läßt sich UV/Vis-spektroskopisch verfolgen. Nach 8 h sind die für das Edukt charakteristischen Banden vollständig verschwunden, und es ist nur noch die sehr breite, langwellig verschobene Absorption des Produktes vorhanden. Die gelbbraune Reaktionslösung wird in 200 ml dest. Wasser eingerührt, auf insgesamt 300 ml mit dest. Wasser verdünnt und mit 100 ml konz. HCI versetzt. Hierbei fällt das Reaktionsprodukt als oranger Niederschlag an. Zur Vervollständigung der Fällung wird eine Stunde bei Raumtemperatur gerührt. Das Reaktionsprodukt wird über eine D4-Glasfilterfritte abgsaugt und 8 h bei 110°C getrocknet. Das erhaltene rote Pulver wird mit 20 ml Eisessig aufgekocht, erneut mit 200 ml dest. Wasser versetzt und mit Kaliumhydroxyd neutralisiert. Hierbei bildet sich ein flockiger, gelber Niederschlag der abgesaugt und 8 h bei 110 °C getrocknet wird. Rohprodukt: Ausb. 150 mg feines, gelbes Pulver. Das Produkt wird vollständig in Chloroform gelöst und säulenchromatographisch an Kieselgel mit Chloroform als Laufmittel gereinigt. Ausb. 130 mg (68 %) feines, oranges Pulver - Schmp. 211°C - R_{f}(CHCl₃) = 0.88. -UV(CHCl₃): λₘₐₓ(ε) = 432.2 (7350), 361.1 (6790), 343.4 (5720), 310.2 (6550), 264.7 (22790). - Fluoreszenz (CHCl₃): λₘₐₓ = 559.3 nm.

| | | | | |
|---|---|---|---|---|
| C₃₅H₃₄N₂O₃ (530.7) | Ber. | C 79.22 | H 6.46 | N 5.28 |
| | Gef. | C 76.87 | H 6.27 | N 5.36 |

### Beispiel 17: N,N'-Di(2-methyl-5-tert-butylphenyl)-1-aminonaphthalin-4,5:8-tricarbonsäure-1,8-lactam-4,5-imid

190 mg (0.34 mmol) des nach Beispiel 8 oben hergestellten N,N'-Di(2-methyl-5-*tert*-butylphenyl)naphthalin-1,8:4,5-tetracarbonsäure-bis(dicarboximid) werden mit 700 mg (12.5 mmol) 85 proz. Kaliumhydroxyd (Plätzchen) in 6 ml Methanol und 4 ml DMSO 3 h auf 100°C erhitzt. Die anfänglich schwach gelbe Lösung verfärbt sich über Gelb nach Orange, Rotbraun und bei Erreichen des Siedepunktes nach Braunschwarz. Die nach beendeter Reaktionszeit hellrote Lösung wird in 200 ml dest. Wasser eingerührt, auf insgesamt 300 ml mit dest. Wasser verdünnt und mit 100 ml konz. HCI versetzt. Hierbei fällt das Reaktionsprodukt als oranger Niederschlag an. Zur Vervollständigung der Fällung wird eine Stunde bei Raumtemperatur gerührt. Das Reaktionsprodukt wird über eine D4-Glasfilterfritte abgesaugt und 8 h bei 110°C getrocknet. Das erhaltene rote Pulver wird in 100 ml Ethanol gelöst, mit 15 ml konz. HCI versetzt und zum Sieden erhitzt, bis der Farbumschlag von Hellrot nach Gelborange erfolgt. Nach Zusatz von 400 ml dest. Wasser bildet sich ein flockiger, gelber Niederschlag der abgesaugt und 8 h bei 110 °C getrocknet wird. Das Produkt wird vollständig in Chloroform gelöst und säulenchromatographisch an aktiviertem Kieselgel (Akt. II, 10 Gew% H₂O) mit Chloroform als Laufmittel gereinigt. Ausb. 170 mg (89%) feines oranges Pulver - Schmp. 211°C - R_{f}(CHCl₃) = 0.88. - UV(CHCl₃): λₘₐₓ(ε) = 432.2 (7350), 361.1 (6790), 343.4 (5720), 310.2 (6550), 264.7 (22790). - Fluoreszenz (CHCl₃): λₘₐₓ = 559.3 nm.

| | | | | |
|---|---|---|---|---|
| C₃₅H₃₄N₂O₃ (530.7) | Ber. | C 79.22 | H 6.46 | N 5.28 |
| | Gef. | C 76.87 | H 6.27 | N 5.36 |

### Beispiel 18: N,N'-Di(1-n-propylcyclohexylmethyl)-1-aminonaphthalin-4,5:8-tricarbonsäure-1,8-lactam-4,5-imid

270 mg (0.45 mmol) des nach Beispiel 6 hergestellten N,N'-Di(1-cyclohexy(-1-propylmethyl)naphthalin-1,8:4,5-tetracarbonsäurebis(dicarboximid) werden mit 290 mg (4.39 mmol) 85 proz. Kaliumhydroxyd-Plätzchen in 40 ml Ethanol 18 h zum Rückfluß erhitzt. Die anfänglich schwach gelbe Lösung verfärbt sich über Gelb nach Orange, Rotbraun und bei Erreichen des Siedepunktes nach Braunschwarz. Die Reaktion läßt sich UV/VIS-spektroskopisch verfolgen. Nach 18 h sind die für das Edukt charakteristischen Banden vollständig verschwunden, und es ist nur noch die sehr breite, langwellig verschobene Absorption des Produktes vorhanden. Die hellrote Reaktionslösung wird in 200 ml dest. Wasser eingerührt, auf insgesamt 300 ml mit dest. Wasser verdünnt und mit 100 ml konz. HCI versetzt. Hierbei fällt das Reaktionsprodukt als tieforanger Niederschlag an. Zur Vervollständigung der Fällung wird eine Stunde bei Raumtemperatur gerührt. Das Reaktionsprodukt wird über eine D4-Glasfilterfritte von der Reaktionslösung abgetrennt und 8 h bei 110°C getrocknet. Das Produkt wird mit 100 ml Eisessig aufgekocht, erneut mit dest. Wasser gefällt, abgesaugt und 8 h bei 110 °C getrocknet. Das Produkt wird vollständig in Chloroform gelöst und mehrmals säulenchromatographisch an Kieselgel mit Chloroform als Laufmittel gereinigt. Ausb. 25 mg (10%) feines, gelboranges Pulver (enthält noch geringe Anteile an Bisimid) - Schmp. 187-191°C - R_{f}(CHCl₃) = 0.91. - UV(CHCl₃): λₘₐₓ = 435.7, 381.6, 361.9, 342.4, 316.7, Sh 268.4. - Fluoreszenz (CHCl₃): λₘₐₓ = 541.5 nm. - MS (70 eV): m/z (%) = 514.3 (100) [M⁺], 497.3 (9) [M⁺-OH], 390.2 (39) [M⁺-C₉H₁₆], 266.1 (62) [M⁺-C₉H₁₆].

### Beispiel 19: N,N'-Di(2,2-dimethylheptyl)-1-aminonaphthalin-4,5:8-tricarbonsäure-1,8-lactam-4,5-imid

270 mg (0.45 mmol) des nach Beispiel 3 oben hergestellten N,N'-Di(2,2-dimethylheptyl)-naphthalin-1,8:4,5-bis(dicarboximid) werden mit 290 mg (4.39 mmol) 85 proz. Kaliumhydroxyd-Plätzchen in 40 ml Ethanol 8 h zum Rückfluß erhitzt. Die anfänglich schwach gelbe Lösung verfärbt sich über Gelb nach Orange, Rotbraun und bei Erreichen des Siedepunktes nach Braunschwarz. Die Reaktion läßt sich UVNIS-spektroskopisch verfolgen. Nach 8 h sind die für das Edukt charakteristischen Banden vollständig verschwunden, und es ist nur noch die sehr breite, langwellig verschobene Absorption des Produktes vorhanden. Die hellrote Reaktionslösung wird in 200 ml dest. Wasser eingerührt, auf insgesamt 300 ml mit dest. Wasser verdünnt und mit 100 ml konz. HCI versetzt. Hierbei fällt das Reaktionsprodukt als tieforanger Niederschlag an. Zur Vervollständigung der Fällung wird eine Stunde bei Raumtemperatur gerührt. Das Reaktionsprodukt wird über eine D4-Glasfilterfritte von der Reaktionslösung abgetrennt und 8 h bei 110°C getrocknet. Das Produkt wird mit 100 ml Eisessig aufgekocht, erneut mit dest. Wasser gefällt, abgesaugt und 8 h bei 110 °C getrocknet. Das Produkt wird vollständig in Chloroform gelöst und säulenchromatographisch an Kieselgel mit Chloroform als Laufmittel gereinigt. Ausb. 22 mg (8%) feines, oranges Pulver (enthält noch geringe Anteile an Bisimid) - Schmp. 94°C - R_{f}(CHCl₃) = 0.89. - UV(CHCl₃): λₘₐₓ (ε) = 439.0 (6220), 361.8 (5170), 343.4 (5480), 328.0 (6380), 311.7 (6790), 267.8 (21320). Fluoreszenz (CHCl₃): λₘₐₓ = 551.0 nm. - MS (70 eV): m/z (%) = 490.3 (100) [M⁺], 473.4 (5) [M⁺-OH], 377.2 (29) [M⁺-C₈H₁₆], 265.1 ( ) [377.2 - C₈H₁₆], 250.0 (1), 235.0 (1).

### Beispiel 20: N,N'-Di(2,2-dimethylheptyl)-1-aminonaphthalin-4,5:8-tricarbonsäure-1,8-lactam-4,5-imid - Umsetzung mit KOH in Methanol/DMSO

260 mg (0.5 mmol) des nach Beispiel 3 oben hergestellten N,N'-Di(2,2-dimethylheptyl)-naphthalin-1,8:4,5-tetracarbonsäurebis(dicarboximid) werden mit 700 mg (12.5 mmol) 85 proz. Kaliumhydroxyd-Plätzchen in einer Mischung aus 4 ml DMSO und 6 ml Methanol 3 h zum Sieden erhitzt. Die anfänglich schwach gelbe Lösung verfärbt sich über Gelb nach Orange, Rotbraun und bei Erreichen des Siedepunktes nach Hellrot. Die hellrote Reaktionslösung wird in 200 ml dest. Wasser eingerührt, auf insgesamt 300 ml mit dest. Wasser verdünnt und mit 100 ml konz. HCI versetzt. Hierbei fällt das Reaktionsprodukt als tieforanger Niederschlag an. Zur Vervollständigung der Fällung wird eine Stunde bei Raumtemperatur gerührt und über Nacht stehengelassen. Das gelborange Reaktionsprodukt wird über eine D4-Glasfilterfritte abgesaugt und 8 h bei 80°C getrocknet (*Achtung*: Das Rohprodukt schmilzt unter 100°C). Das Produkt wird vollständig in Chloroform gelöst und säulenchromatographisch an Kieselgel (fein) mit Chloroform als Laufmittel gereinigt. Nach Entfernen des Lösungsmittels erhält man 50 mg (20 %) gelbes Pulver, das aus Ethanol/Wasser umkristallisiert wird. Ausb. 45 mg (18%) feines, gelbes Pulver - Schmp. 97 °C - R_{f}(CHCl₃) = 0.89. - UV(CHCl₃): λₘₐₓ (ε) = 439.0 (6220), 361.8 (5170), 343.4 (5480), 328.0 (6380), 311.7 (6790), 267.8 (21320). - Fluoreszenz (CHCl₃): λₘₐₓ = 551.0 nm. - MS (70 eV): m/z (%) = 490.3 (100) [M⁺], 473.4 (5) [M⁺-OH], 377.2 (29) [M⁺-C₈H₁₆], 265.1 [377.2-C₈H₁₆], 250.0 (1), 235.0 (1).

### Beispiel 21: Umsetzung von N,N'-Di(2-tert-butylphenyl)naphthalin-1,8:4,5-bis(dicarboximid) zum Lactam-imid - unter O₂-Ausschluß

200 mg (0.38 mmol) des nach Beispiel 9 oben hergestellten Bis(dicarboximid) werden mit 200 mg 85 proz. KOH und 20 ml Ethanol in einem mit Argon befüllten Kolben suspendiert und im N₂-Strom während 3 h zum Sieden erhitzt. Hierbei beobachtet man mehrere Farbwechsel der Reaktionsmischung: von Gelb nach Gelbbraun über Braun nach Rotviolett. Nach beendeter Reaktionszeit werden 200 ml dest. Wasser (Farbumschlag nach Gelborange) und 100 ml konz. HCI zugegeben und 1 h bei Raumtemperatur gerührt. Man läßt über Nacht stehen, trennt den erhaltenen, gelben Niederschlag über eine D4-Glasfilter-fritte ab und trocknet 2 h bei 110°C. Das Rohprodukt (170 mg gelboranges Pulver) wird zweimal mit je 200 ml 10 proz. Kaliumcarbonatlösung aufgekocht. Der unlösliche Rückstand (125 mg gelbes Pulver: Gemisch A) wird abgetrennt und das gelb gefärbte Filtrat vorsichtig mit konz. HCI neutralisiert. Das aus dem alkalischen Filtrat ausgefällte Produkt wird ebenfalls abgetrennt und getrocknet. Man erhält 45 mg oranges Pulver (Gemisch B). Beide Rohprodukte werden NMR- und massenspektroskopisch untersucht. Massenspektroskopisch lassen sich im Gemisch A zwei Komponenten nachweisen, neben nicht umgesetztem Edukt ist das gewünschte Lactam enthalten. Gemisch B besteht aus drei Verbindungen, Edukt, Lactam und dem Produkt der partiellen Verseifung, dem N-(2-*tert*-Butylphenyl)naphthalin-1,8:4,5-tetracarbonsäure-1,8-imid-4,5-anhydrid. Aus dem ¹H-NMR-Spektren beider Rohprodukte lassen sich die Mengen der enthaltenen Komponenten grob abschätzen. Das nicht umgesetzte Edukt liefert ein Singulett bei 8.85 ppm für die vier Wasserstoffatome des Naphthalin-Grundgerüstes, für das Lactam findet man vier verschiedene Dubletts bei 8.70, 8.43, 8.31 und 6.75 ppm, jeweils einem Wasserstoffatom entsprechend, und dem Verseifungsprodukt (N-(2-*tert*-Butylphenyl)naphthalin-1,8:4,5-tetracarbonsäure-1,8-imid-4,5-anhydrid) entsprechen zwei Dubletts bei 8.62 und 7.87 ppm.
Gemisch A (125 mg): 60 % Edukt : 40 % Lactam. - MS (70 eV): m/z (%) = 530.2 (0.5) [M⁺, Edukt], 515.2 (4) [M⁺-CH₃, Edukt], 473.1 (100) [M⁺-C(CH₃)₃, Edukt], 445.1 (84) [M⁺-C(CH₃)₃, Lactam], 416.1 (8) [473-C(CH₃)₃, Edukt], 342.0 (30) [M⁺-C(CH₃)₃, Monoimid-monoanhydrid]. Gemisch B (45 mg): 47 % Edukt : 29 % Lactam : 24 % Monoimid-monoanhydrid. - MS (70 eV): m/z (%) = 530.2 (0.5) [M⁺, Edukt], 515.2 (2) [M⁺-CH₃, Edukt], 473.1 (56) [M⁺-C(CH₃)₃, Edukt], 463.1(10), 445.1 (56) [M⁺-C(CH₃)₃, Lactam], 416.1 (9) [473-C(CH₃)₃, Edukt], 384.1 (6), 371.1 (15), 366.1 (10), 356.1 (9), 354.1 (8), 342.0 (100) [M⁺-C(CH₃)₃, Monoimid-monoanhydrid], 338.1 (8).
Folgende Ausbeuten der Komponenten der beiden Gemische werden erhalten:
- 10 mg (50%) N,N'-Di(2-*tert*-butylphenyl)naphthalin-1,8:4,5-bis(dicarboximid) (Ausgangsmaterial)
- 60 mg (31%) N-(2-*tert*-Butylphenyl)-1-aminonaphthalin-8:4,5-tricarbonsäure-1,8-lactam-4,5-imid
- 10 mg (6.6%) N-(2-*tert*-Butylphenyl)naphthalin-1,8:4,5-tetracarbonsäure-1,8-imid-4,5-anhydrid (partielle Verseifung).

## Patentansprüche

1. Naphthalin-Lactam-Imide der Formel I 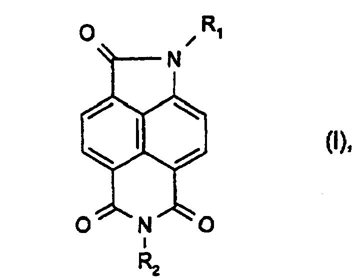 worin R₁ und R₂ gleich sind, und eine verzweigte unsubstituierte C₃-C₂₅-Alkylgruppe, eine C₂-C₂₅-Alkylgruppe, die mit C₃-C₁₀-Cycloalkyl substituiert ist, oder einen Rest der Formel II 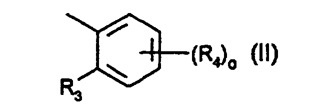 bedeuten,
worin R₃ und R₄ C₁-C₁₂-Alkyl sind, und o 0 oder 1 ist.

2. Naphthalin-Lactam-lmide nach Anspruch 1, worin R₁ und R₂ einen Rest der Formel II mit R₃ und R₄ unabhängig voneinander gleich Methyl oder tert.-Butyl, vorzugsweise 2-Methylphenyl, 2,3-Dimethylphenyl, 2,5-Dimethylphenyl, 2-Methyl-5-tert.butylphenyl-, 2-tert.Butylphenyl, 2,3-Di-tert.butylphenyl oder 2,5-Di-tert.butylphenyl bedeuten.

3. Naphthalin-Lactam-Imide nach Anspruch 1, worin R₁ und R₂ 2-Butyl, 2-Methyl-2-butylhexyl, 2,2-Dimethylheptyl, 2,2-Dihexyloctyl-, 1-Ethylcyclohexylmethyl oder 1-(n-Propyl)-cycloohexylmethyl bedeuten.

4. Naphthalin-Lactam-lmide nach Anspruch 1, worin R₁ und R₂ -CH(R₈)₂ mit R₈ gleich geradkettiges C₁-C₁₂-Alkyl, vorzugsweise n-Hexyl, n-Heptyl, n-Octyl, n-Nonyl oder n-Decyl sind.

5. Napthalintetracarbonsäure-Bisimide der Formel V 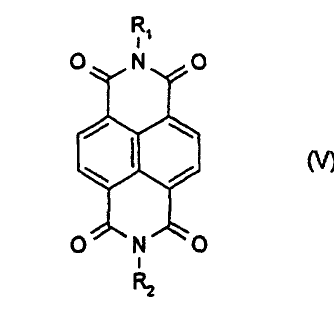 worin R₁ und R₂ die in Anspruch 1 angegebene Bedeutung haben, mit der Massgabe, dass R₁ und R₂ nicht beide Ethyl oder Decyl sind.

6. 1-Aminonaphthalintricarbonsäurederivate der Formel VI 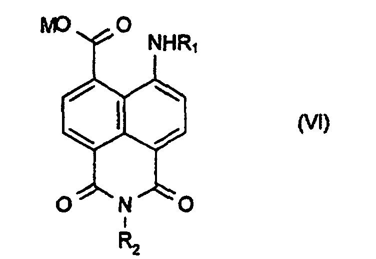 worin R₁ und R₂ die in Anspruch 1 angegebene Bedeutung haben, und M für Wasserstoff oder ein Alkalimetallkation, insbesondere für Wasserstoff oder Kalium, steht.

7. Verfahren zur Herstellung von Naphthalin-Lactam-Imiden nach Anspruch 1, durch Umsetzung eines Napthalintetracarbonsäure-Bisimids nach Anspruch 5 mit einer Base.

8. Verfahren nach Anspruch 7, worin die Umsetzung in einem organischen Lösungs-mittel, vorzugsweise in Methanol oder Ethanol, durchgeführt wird, und die Base ein Alkalimetallhydroxid, vorzugsweise Kaliumhydroxid, ist.

9. Verfahren nach Anspruch 7 oder 8, worin das Lösungsmittel zusätzlich Dimethyl-sulfoxid, vorzugsweise 30-50 Vol.-% Dimethylsulfoxid, enthält.

10. In der Masse eingefärbtes hochmolekulares organisches Material enthaltend eine Verbindung nach einem der Ansprüche 1 bis 5.

11. Verfahren zum Färben von hochmolekularem organischem Material in der Masse, **gekennzeichnet durch** Verwendung einer Verbindung nach einem der Ansprüche 1 bis 5.

12. Verwendung einer Verbindung nach einem der Ansprüche 1 bis 5 im Sicherheitsdruck, als Fluoreszenzfarbstoffe für maschinenlesbare Markierungen, als Laserfarbstoffe, sowie für die Herstellung von Druck-Tonern ("non-impact printing toners"), Farbfiltern, organischen Photorezeptoren, Elektrolumineszenz- und Photolumineszenzelementen, optischen Informationsspeichermedien oder Sonnenkollektoren.

## Claims

1. A naphthalenelactamimide of the formula I 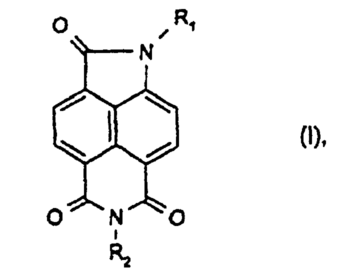 where R₁ and R₂ are the same and are a branched unsubstituted C₃-C₂₅alkyl group, a group which is substituted by C₃-C₁₀cycloalkyl or a radical of the formula II 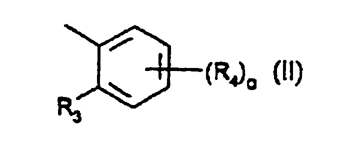 in which R₃ and R₄ are C₁-C₁₂alkyl, and o is 0 or 1.

2. A naphthalenlactamimide according to claim 1, in which R₁ and R₂ are a radical of the formula II, where R₃ and R₄ independently of one another are methyl or tert-butyl, preferably 2-methylphenyl, 2,3-dimethylphenyl, 2,5-dimethylphenyl, 2-methyl5-tert-butylphenyl-, 2-tert-butylphenyl, 2,3-di-tert-butylphenyl or 2,5-di-tert-butylphenyl.

3. A naphthalenelactamimide according to claim 1, in which R₁ and R₂ are 2-butyl, 2-methyl-2-butylhexyl, 2,2-dimethylheptyl, 2,2-dihexyloctyl, 1-ethylcyclohexylmethyl or 1-(n-propyl)-cyclohexylmethyl.

4. A naphthalenelactamimide according to claim 1, in which R₁ and R₂ are -CH(R₈)₂ where R₈ is straight-chain C₁-C₁₂alkyl, preferably n-hexyl, n-heptyl, n-octyl, n-nonyl or n-decyl.

5. A napthalenetetracarboxylic acid bisimide of the formula V 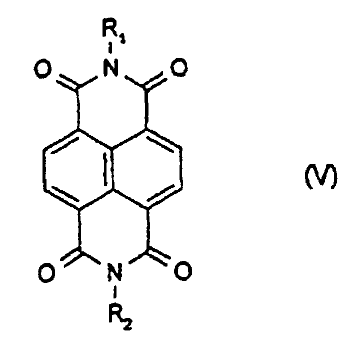 in which R₁, and R₂ are as defined in claim 1, with the proviso that R₁ and R₂ are not both ethyl or decyl.

6. A l-aminonaphthalinetricarboxylic acid derivative of formula VI 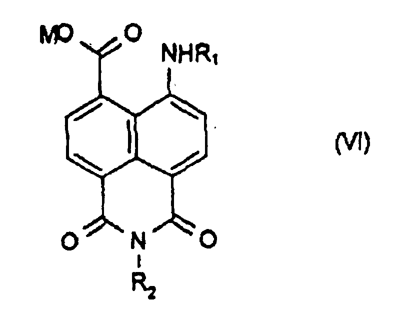 in which R₁ and R₂ are as defined in claim 1 and M represents hydrogen or an alkali metal cation, in particular hydrogen or potassium.

7. A process for the preparation of a naphthalenelactamimide according to claim 1, by reaction of a naphthalenetetracarboxylic acid bisimide according to claim 5 with a base.

8. A process according to claim 7, wherein the reaction is carried out in an organic solvent, preferably in methanol or ethanol, and the base is an alkali metal hydroxide, preferably potassium hydroxide.

9. A process according to claim 7 or 8, wherein the solvent additionally comprises dimethyl sulfoxide, preferably 30-50% by volume of dimethyl sulfoxide.

10. A mass-coloured high molecular weight organic material comprising a compound according to any one of claims 1 to 5.

11. A process for mass-colouring a high molecular weight organic material, which comprises using a compound according to any one of claims 1 to 5.

12. The use of a compound according to any one of claims 1 to 5 in security printing, as a fluorescence dye for machine-readable markings, as a laser dye and for the preparation of printing toners ("non-impact printing toners"), colour filters, organic photoreceptors, electroluminescent and photoluminescent elements, optical information storage media or solar collectors.

## Revendications

1. Naphtalène-lactamimides de formule I 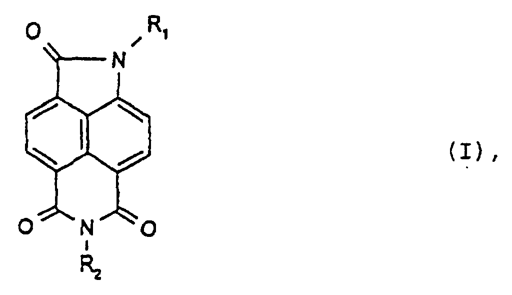 où R₁ et R₂ sont identiques et représentent un groupe alkyle en C₃-C₂₅ ramifié, non substitué, un groupe alkyle en C₂-C₂₅ qui est substitué par un groupe cycloalkyle en C₃-C₁₀, ou un reste de formule II 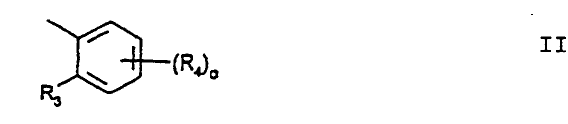 où
R₃ et R₄ représentent un groupe alkyle en C₁-C₁₂ et o vaut 0 ou 1.

2. Naphtalène-lactamimides selon la revendication 1, où R₁ et R₂ représentent un reste de formule II avec R³ et R⁴ représentant, indépendamment l'un de l'autre, un groupe méthyle ou tert.-butyle, de préférence 2-méthylphényle, 2,3-diméthylphényle, 2,5-diméthylphényle, 2-méthyl-5-tert.-butylphényle, 2-tert.-butylphényle, 2,3-di-tert.-butylphényle ou 2,5-di-tert.-butylphényle.

3. Naphtalène-lactamimides selon la revendication 1, où R₁ et R₂ représentent des groupes 2-butyle, 2-méthyl-2-butylhexyle, 2,2-diméthylheptyle, 2,2-dihexyloctyle, 1-éthylcyclohexylméthyle ou 1-(n-propyl)-cyclohexylméthyle.

4. Naphtalène-lactamimides selon la revendication 1, où R₁ et R₂ représentent un groupe -CH(R₈)₂ où R₈ représente un groupe alkyle en C₁-C₁₂ linéaire, de préférence n-hexyle, n-heptyle, n-octyle, n-nonyle ou n-décyle.

5. Naphtalènetétracarboxy-bisimides de formule V 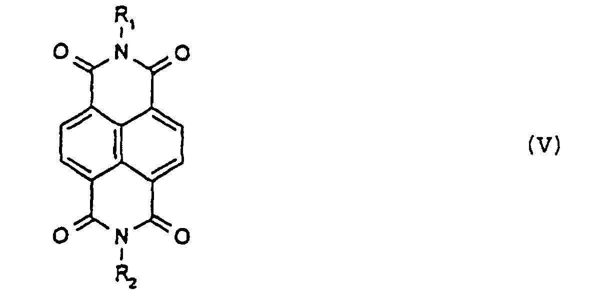 où R₁ et R₂ possèdent la signification donnée à la revendication 1, à condition que R₁ et R₂, les deux, ne représentent pas éthyle ou décyle.

6. Dérivés de l'acide 1-aminonaphtalènetricarboxylique de formule VI 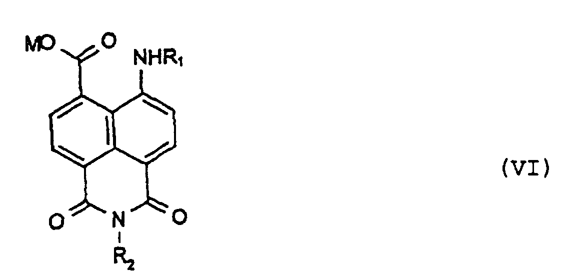 où R₁ et R₂ possèdent les significations données à la revendication 1, et M représente un atome d'hydrogène ou un ion métal alcalin, en particulier un atome d'hydrogène ou de potassium.

7. Procédé pour la préparation de naphtalène-lactamimides selon la revendication 1, par transformation d'un naphtalène-tétracarboxybisimide selon la revendication 5 sur une base.

8. Procédé selon la revendication 7, où on met en oeuvre la réaction dans un solvant organique, de préférence le méthanol ou l'éthanol, et la base est un hydroxyde de métal alcalin, de préférence l'hydroxyde de potassium..

9. Procédé selon la revendication 7 ou 8, où le solvant contient de plus du diméthylsulfoxyde, de préférence de 30 à 50 % vol. de diméthylsulfoxyde.

10. Matière organique de haut poids moléculaire colorée en masse contenant un composé selon l'une des revendications 1 à 5.

11. Procédé pour la coloration dans la masse de matières organiques de haut poids moléculaire, **caractérisé par** l'utilisation d'un composé selon l'une des revendications 1 à 5.

12. Utilisation d'un composé selon l'une des revendications 1 à 5 dans l'impression de signalisation de sécurité, en tant que colorants fluorescents pour des marquages lisibles par machine, en tant que laser au colorants, ainsi que pour la préparation de toners d'impression ("non-impact printing toners"), de films couleur, de photorécepteurs organiques, d'éléments électroluminescents et photolumiescents, de milieu de stockage d'informations optiques ou de collecteurs d'énergie solaire.
